# EUROPÄISCHE PATENTSCHRIFT

(11) **EP 3 566 666 B1**
(45) Veröffentlichungstag und Bekanntmachung des Hinweises auf die Patenterteilung: **30.08.2023**
(21) Anmeldenummer: 19173570.3
(22) Anmeldetag: 09.05.2019
(51) Int. Cl.: A61B 17/92

(54) **MEDIZINISCHES WERKZEUG MIT EINEM ZUGFESTEN HEFT UND VERFAHREN ZUR HERSTELLUNG EINES SOLCHEN WERKZEUGS**
MEDICAL TOOL WITH A HANDLE WITH HIGH TENSILE STRENGTH AND METHOD FOR PRODUCING SUCH A TOOL
OUTIL MÉDICAL COMPORTANT UN MANCHE RÉSISTANT À LA TRACTION ET PROCÉDÉ DE FABRICATION D'UN TEL OUTIL

(30) Priorität: 09.05.2018 DE 102018111225
(43) Veröffentlichungstag der Anmeldung: 13.11.2019
(73) Patentinhaber: Aesculap AG, 78532 Tuttlingen (DE)
(72) Erfinder: DAHMEN, Jan, 78606 Seitingen-Oberflacht (DE)
(74) Vertreter: Winter, Brandl - Partnerschaft mbB

(56) Entgegenhaltungen:
- DE-A1-102016 112 788
- DE-U- 1 531 079
- US-A1- 2018 028 224

## Beschreibung

### Technisches Gebiet

Die vorliegende Erfindung bezieht sich auf ein medizinisches Werkzeug mit einem Arbeitsteil und einem Werkzeugheft sowie auf ein Verfahren zur Herstellung eines solchen Werkzeugs.

### Stand der Technik

Es ist bekannt, einen klinischen Hammerkopf mit einem zuvor aus zwei Hohlhefthälften hergestellten Hammerheft bzw. Hammergriff zu verbinden. Damit sich die Verbindung zwischen Hammerheft und Hammerkopf gut für die Übertragung von Momenten eignet, kann ein axial verlaufender Abschnitt des Hammerkopfs von dem Hammerkopf umschlossen sein.

Das Problem derartig hergestellter klinischer Hämmer ist, dass bei einem vollständigen oder unvollständigen Bruch der Schweißnaht zwischen Werkzeugheft und Arbeitsteil eine erhöhte Gefahr für ein Lösen des Arbeitsteils besteht und somit diese Werkzeuge im Falle von Hämmern für den klinischen Einsatz ein erhöhtes Patientenrisiko darstellen. Um solch ein Patientenrisiko zu verkleinern, ist es bei Hämmern für den klinischen Einsatz üblich, einen Stift vorzusehen, welcher das entsprechende Hammerheft mit dem entsprechenden Hammerkopf zusätzlich verbindet und somit die Verbindung zwischen diesen beiden Teilen formschlüssig sichert. Solche Sicherungen durch Stifte erhöhen allerdings den Herstellungsaufwand und die Herstellungskosten.

In DE 10 2016 112 788 A1 ist ein medizinisches Werkzeug offenbart, bei dem ein Griffkern mit einem Handgriff stoffschlüssig verbunden ist und bei dem der Griffkern zusätzlich einen Abschlussdeckel aufweist, der formschlüssig mit dem Handgriff zusammenwirkt.

In DE 15 31 079 U ist ein Hammer mit einem Stahlrohrstiel offenbart. Der Stahlrohrstiel ist auf ein mit einem Hammerkopf einstückig ausgebildeten Ansatzstück aufgesteckt. Die Verbindung zwischen dem Stahlrohrstiel und dem Hammerkopf ist über Rastkerben im Ansatzstück und über Schweißnähte zwischen Stahlrohrstiel und Hammerkopf bewerkstelligt.

Aufgabe der vorliegenden Erfindung ist es deshalb, ein medizinisches Werkzeug, insbesondere in Form eines Hammers, bereitzustellen, bei welchem die Verbindung zwischen einem Werkzeugheft und einem Arbeitsteil kostengünstig verbessert ist.

Diese Aufgabe wird durch ein medizinisches Werkzeug gemäß Anspruch 1 gelöst, bei dem ein Werkzeugheft form- und stoffschlüssig mit einem Arbeitsteil verbunden ist. Ein Verfahren gemäß Anspruch 9 zur Herstellung eines solchen Werkzeugs löst ebenfalls die Aufgabe. Vorteilhafte Weiterbildungen sind Gegenstand der Unteransprüche.

Gemäß der vorliegenden Erfindung weist ein medizinisches Werkzeug, insbesondere in Form eines klinischen Hammers, ein Werkzeugheft, welcher auch Werkzeuggriff genannt werden kann, und ein Arbeitsteil auf. Das Arbeitsteil ist abschnittsweise in dem Werkzeugheft aufgenommen und mit diesem stoffschlüssig, insbesondere über eine Schweißverbindung, verbunden.

Ein in dem Werkzeugheft aufgenommener Abschnitt des Arbeitsteils, welcher auch Erl oder Angel genannt werden kann, weist eine Aufweitung auf, welche zumindest im Falle eines Lösens des Stoffschlusses zwischen Werkzeugheft und Arbeitsteil zur Axialsicherung (in zumindest einer distalen Richtung) einen axialen Formschluss mit einer Verengung (innerhalb) des Werkzeughefts eingeht.

Das Arbeitsteil ist in einer Haupterstreckungsachse des Werkzeughefts distal vom Werkzeugheft angeordnet. An einem distalen Ende weist das Arbeitsteil einen Funktionsabschnitt auf, beispielsweise einen Hammerkopf oder eine Klinge. Abseits dieses Funktionsabschnitts, vorzugsweise in einem Bereich eines proximalen Endes, weist das Arbeitsteil einen axial verlaufenden Abschnitt auf, welcher mit (zumindest) einem radial vorragenden Vorsprung versehen ist. Dieser Abschnitt ist in einer axial verlaufenden Aussparung in dem Werkzeugheft bzw. innerhalb des Werkzeughefts aufgenommen, so dass der Vorsprung proximal von einem distalen Ende des Werkzeughefts mit einer Umwandung, einem radialen Rücksprung oder einem Rand der Aussparung formschlüssig zusammenwirken kann. Der Vorsprung ragt vorzugsweise in alle radialen Richtungen vor bzw. ist komplett umlaufend vorgesehen.

Der Vorteil des erfindungsgemäßen medizinischen Werkzeugs ist, dass bei Lösen oder Brechen des Stoffschlusses zwischen Werkzeugheft und Arbeitsteil die Verbindung zwischen diesen beiden Teilen weiterhin zumindest behelfsmäßig mittels des Formschlusses gewährleistet werden kann.

Das Werkzeugheft weist zumindest zwei miteinander verbundene Heftteile auf und der in dem Werkzeugheft aufgenommene Abschnitt des Arbeitsteils ist zwischen den zumindest zwei Heftteilen aufgenommen.

Weisen die Bereiche des Arbeitsteils abseits des in dem Werkzeugheft geführten Abschnitts, insbesondere in dem Funktionsabschnitt, keine Querschnitte auf, die größer sind als der Querschnitt des Werkzeughefts, kann die Aussparung in dem Werkzeugheft in Form einer Durchgangsöffnung vorgesehen sein, durch welche das Arbeitsteil mit dem Funktionsabschnitt voran eingeführt werden kann, bis die Aufweitung an der Verengung anschlägt. Nach Einführen des Arbeitsteils in das Werkzeugheft wird das Arbeitsteil dann, insbesondere bezüglich der Verengung jenseits der Aufweitung, stoffschlüssig mit dem Werkzeugheft verbunden.

Weisen die Bereiche des Arbeitsteils abseits des in dem Werkzeugheft geführten Abschnitts jedoch größere Querschnitte auf, beispielsweise im Falle eines Hammerkopfs, ist es vorteilhaft, das Werkzeugheft zumindest zweiteilig auszugestalten, und zwischen diesen zumindest zwei Heftteilen einen zwischen der Aufweitung und dem Funktionsabschnitt angeordneten Bereich des Arbeitsteils aufzunehmen.

Die zumindest zwei Heftteile des medizinischen Werkzeugs können miteinander stoffschlüssig, insbesondere über eine Schweißverbindung, verbunden sein. Durch diesen Stoffschluss ist es in vorteilhafter Weise möglich, Fugen zu vermeiden und somit die bei einer Sterilisierung des medizinischen Werkzeugs zu reinigende Oberfläche zu verringern.

Um die Herstellungskosten zu verringern, kann das medizinische Werkzeug in vorteilhafter Weise so ausgebildet werden, dass in einem Abschnitt des medizinischen Werkzeugs der Stoffschluss zwischen den zumindest zwei Heftteilen gleichzeitig mit dem Stoffschluss zwischen Werkzeugheft und Arbeitsteil gewährleistet wird.

Der in dem Werkzeugheft aufgenommene Abschnitt des Arbeitsteils kann zumindest teilweise nicht rotationssymmetrisch sein. Vorzugsweise kann dieser Abschnitt einen elliptischen Querschnitt aufweisen. Durch diese fehlende Rotationssymmetrie ist es möglich, durch entsprechende nichtrotationssymmetrische Gestaltung der Innenwände der axialen Aussparung des Werkzeughefts eine formschlüssige Drehsicherung gegen ein Verdrehen des Werkzeughefts bezüglich des Arbeitsteils in Umfangsrichtung bezüglich der Haupterstreckungsachse des Werkzeughefts zu gewährleisten. Insbesondere ist es möglich, die Aufweitung des Arbeitsteils und die Verengung des Werkzeuggriffs entsprechend aufeinander abgestimmt nicht-rotationssymmetrisch auszugestalten.

Die Aufweitung des Arbeitsteils kann an einem proximalen Ende des Arbeitsteils vorgesehen sein. Durch Anordnen der Aufweitung an dem proximalen Ende des Arbeitsteils kann in vorteilhafter Weise der Hebel des Arbeitsteils, welcher zur Übertragung eines Moments dient, vergrößert werden.

An dem proximalen Ende des Arbeitsteils kann eine axiale Aushöhlung vorgesehen sein. Die Gewichtsverteilung bekannter medizinischer Werkzeuge ist zuweilen zu grifflastig, so dass die Handhabbarkeit dieser Werkzeuge eingeschränkt sein kann. Durch Vorsehen der axialen Aushöhlung kann in vorteilhafter Weise der Schwerpunkt des Werkzeugs in Richtung des distalen Endes verschoben werden, womit eine ausbalancierte oder auch kopflastige Gewichtsverteilung ermöglicht werden kann.

An einem distalen Ende des in dem Werkzeugheft aufgenommenen Abschnitts des Arbeitsteils kann eine weitere Aufweitung vorgesehen sein, welche einen Absatz bildet, der mit einem distalen Ende des Werkzeughefts stoffschlüssig verbunden ist bzw. umgekehrt. Im Falle eines Lösens des Stoffschlusses zwischen Werkzeugheft und Arbeitsteil kann der Absatz eine zusätzliche Axialsicherung (durch einen Formschluss) gewährleisten, vorzugweise in einer Richtung entgegengesetzt der Richtung, in welche die Aufweitung des in dem Werkzeugheft aufgenommenen Abschnitts des Arbeitsteils axial sichert. Insbesondere kann der Absatz derart ausgestaltet sein, dass eine Außenfläche des Werkzeughefts bündig in eine Außenfläche des Arbeitsteils übergeht. Der Absatz bildet einen axialen Anschlag bzw. eine axiale Sicherung, damit das Arbeitsteil nicht in das Werkzeugheft reinrutscht, wenn sich der Stoffschluss zwischen Arbeitsteil und Werkzeugheft löst. Das heißt, der Absatz sichert nach innen, die Aufweitung des in dem Werkzeugheft aufgenommenen Abschnitts des Arbeitsteils und die Verengung am Werkzeugheft sichern nach außen. Durch den Absatz kann auch auf vorteilhafte Weise das stoffschlüssige Verbinden des Werkzeughefts mit dem Arbeitsteil und somit die Herstellung des Werkzeugs vereinfacht werden.

Zwischen der Verengung des Werkzeughefts und der einem axialen Formschluss dienenden Aufweitung des Arbeitsteils kann ein axiales Spiel vorgesehen sein. Das axiale Spiel hat zur Folge, dass bei einem intakten medizinischen Werkzeug die Verbindung zwischen Werkzeugheft und Arbeitsteil lediglich durch den Stoffschluss zwischen diesen beiden Teilen gewährleistet wird. Erst wenn sich dieser Stoffschluss löst, kommt der Formschluss zwischen der Aufweitung des Arbeitsteils und der Verengung des Werkzeughefts zum Tragen. Die hat zwei Vorteile. Zum einen ist die Erkennung des defekten Zustands des Werkzeugs einfacher, da sich bei einem gebrochenen Stoffschluss zwischen Arbeitsteil und Werkzeugheft das Arbeitsteil relativ zum Werkzeugheft bewegen lässt. Zum anderen kann im Vergleich zu einem medizinischen Werkzeug ohne axialem Spiel die Sicherungsleistung vergrößert werden.

Gegenstand der Erfindung ist auch ein Verfahren zur Herstellung eines medizinischen Werkzeugs mit den Schritten
- formschlüssiges Verbinden eines Werkzeughefts mit einem Arbeitsteil und
- anschließendes stoffschlüssiges Verbinden des Werkzeughefts mit dem Arbeitsteil.

Der Formschluss zwischen Werkzeugheft und Arbeitsteil wird durch Einlegen eines mit einer Aufweitung versehenen Abschnitts des Arbeitsteils zwischen zwei eine Verengung bildende Heftteile des Werkzeughefts bewerkstelligt und daran anschließend werden die zumindest zwei Heftteile miteinander stoffschlüssig verbunden.

Der Stoffschluss zwischen Werkzeugheft und Arbeitsteil und der Stoffschluss zwischen den zumindest zwei Heftteilen können zumindest teilweise gleichzeitig bewirkt werden.

### Kurzbeschreibung der Zeichnungen

Die vorliegende Erfindung wird im Folgenden anhand bevorzugter Ausführungsbeispiele unter Bezugnahme auf die beigefügten Zeichnungen näher beschrieben. Es zeigen:
Fig. 1 eine Seitenansicht eines erfindungsgemäßen medizinischen Werkzeugs in seinen Einzelteilen;
Fig. 2 eine seitliche Teilschnittansicht des erfindungsgemäßen medizinischen Werkzeugs in seinen Einzelteilen;
Fig. 3 eine seitliche Teilschnittansicht eines ersten Zwischenprodukts bei der Herstellung des erfindungsgemäßen medizinischen Werkzeugs;
Fig. 4 eine seitliche Teilschnittansicht eines zweiten Zwischenprodukts bei der Herstellung des erfindungsgemäßen medizinischen Werkzeugs;
Fig. 5 eine Seitenansicht des zweiten Zwischenprodukts bei der Herstellung des erfindungsgemäßen medizinischen Werkzeugs;
Fig. 6 eine Seitenansicht eines dritten Zwischenprodukts bei der Herstellung des erfindungsgemäßen medizinischen Werkzeugs;
Fig. 7 eine Seitenansicht eines vierten Zwischenprodukts bei der Herstellung des erfindungsgemäßen medizinischen Werkzeugs; und
Fig. 8 eine Seitenansicht des erfindungsgemäßen medizinischen Werkzeugs.

Gleiche oder funktional äquivalente Merkmale sind in den einzelnen Figuren mit denselben Bezugszeichen versehen.

### Detaillierte Beschreibung bevorzugter Ausführungsformen

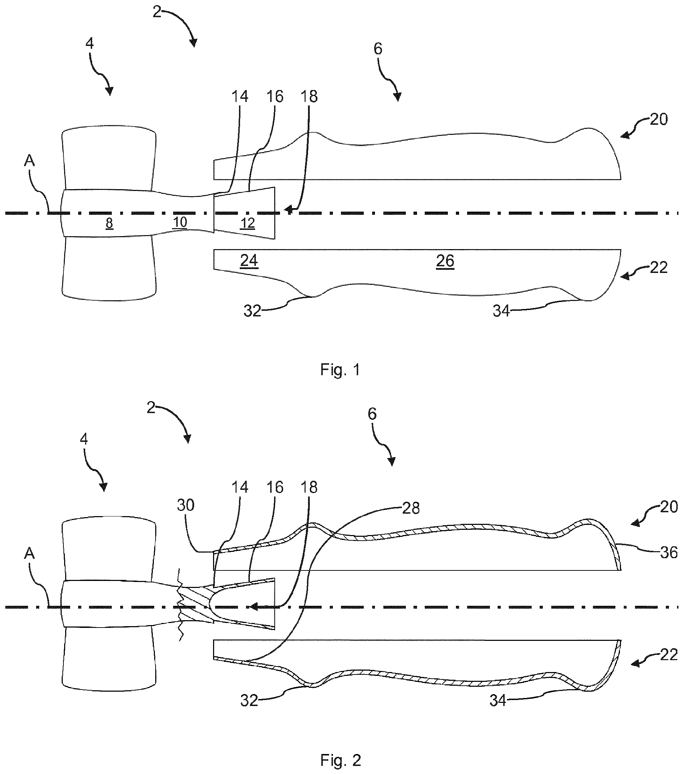

Wie in Fig. 1 gezeigt, weist ein medizinisches Werkzeug 2 gemäß der vorliegenden Erfindung ein Arbeitsteil 4 und ein Werkzeugheft 6 auf.

Das Arbeitsteil 4 weist einen Funktionsabschnitt 8, einen Schaftabschnitt 10 und einen Verbindungsabschnitt 12 auf. In der vorliegenden Ausführungsform ist das Arbeitsteil 4 als ein Hammerkopf ausgebildet, wobei der eigentliche Kopf im Wesentlichen in Form eines Zylinders mit zwei gegenüberliegenden Schlagflächen an einem bezüglich einer Haupterstreckungsachse A des Werkzeugs distalen Ende des Arbeitsteils 4 im Funktionsabschnitt 8 ausgebildet ist. Von diesem eigentlichen Kopf aus erstreckt sich senkrecht zu diesem in eine proximale Richtung der Schaftabschnitt 10. Der Schaftabschnitt 10 ist im Wesentlichen ein Zylinder mit elliptischen Außenausmaß, wobei das elliptische Außenausmaß über die Länge des Schaftabschnitts 10 nicht ganz konstant bleibt, sondern sich von einem distalen Ende des Schaftabschnitts 10 zunächst bis etwa zur Mitte des Schaftabschnitts 10 hin etwas verkleinert bevor er sich in Richtung eines proximalen Ende wieder vergrößert. An dem proximalen Ende des Schaftabschnitts 10 weist das Arbeitsteil 4 einen Absatz 14 auf, bei welchem sich das Außenausmaß des Querschnitts des Arbeitsteils 4 sprunghaft in proximaler Richtung verkleinert. Proximal von diesem Absatz 14 schließt sich der Verbindungsabschnitt 12 an. Der Verbindungsabschnitt 12 ist ein elliptischer Hohlzylinderstumpf, dessen Querschnittsaußenausmaß sich ausgehend vom Absatz 14 in proximaler Richtung vergrößert bzw. aufweitet. Der Verbindungsabschnitt 12 des Arbeitsteils 4 bzw. dessen Außenfläche bildet somit eine Aufweitung 16 des Arbeitsteils 4.

Das Arbeitsteil 4 ist aus Metall hergestellt. Der Funktionsabschnitt 8 ist gänzlich massiv ausgebildet. An einer proximalen Stirnseite des Arbeitsteils 4 ist, wie in Fig. 2 gezeigt, eine Aussparung bzw. Aushöhlung 18 vorgesehen, so dass der Verbindungsabschnitt 12 gänzlich hohl ausgebildet ist. Der Schaftabschnitt 10 ist bis auf einen proximalen Teilabschnitt, in welchem sich die Aushöhlung 18 bis in den Schaftabschnitt 10 hineinerstreckt, wie der Funktionsabschnitt 8 massiv ausgebildet.

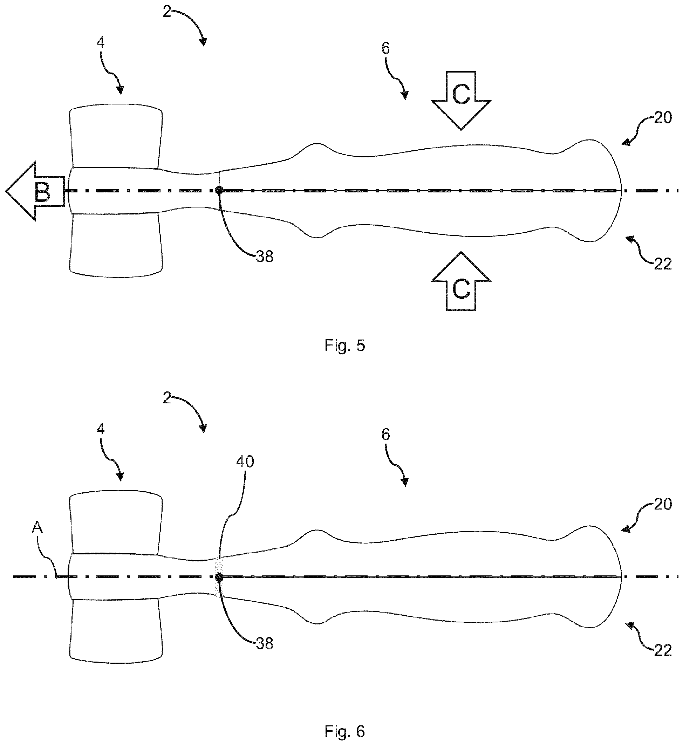

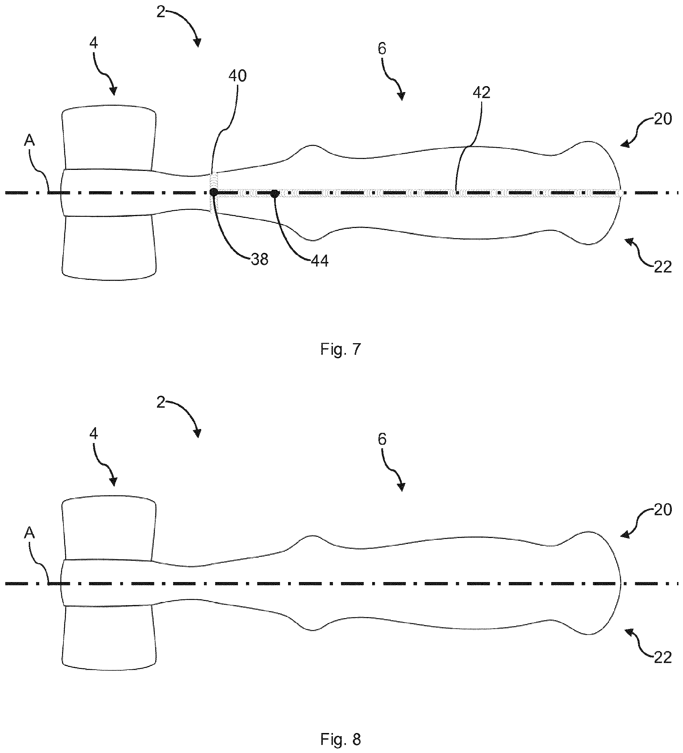

Das Werkzeugheft 6 ist als Hohlkörper ausgebildet, dessen äußere Form der Form eines abgeplatteten Rotationskörpers entspricht, und weist zwei schalen- bzw. wannenförmige Heftteile 20 und 22 auf, welche identisch zueinander ausgebildet sind. Die Trenn- bzw. Verbindungsebene, in welcher die beiden Heftteile 20 und 22 im Endzustand der Herstellung des erfindungsgemäßen Werkzeugs 2 aneinanderstoßen (siehe Figuren 5 und 8), liegt auf der Haupterstreckungsachse A des Werkzeugs 2. Das Querschnittsaußenausmaß des Werkzeughefts 6 ist wie die Querschnittsaußenausmaße des Schaftabschnitts 10 und des Verbindungsabschnitts 12 des Arbeitsteils 4 elliptisch. Die Hauptachsen der entsprechenden Ellipsen sind wie im Schaftabschnitt 10 und im Verbindungsabschnitt 12 parallel zu der Erstreckungsrichtung des Zylinders des Funktionsabschnitts 8. Die Nebenachsen sind parallel zu Verbindungsebene zwischen den Hefteilen 20 und 22.

Das Werkzeugheft 6 weist an einer distalen Seite einen Verbindungsabschnitt 24 und an einer proximalen Seite einen Griffabschnitt 26 auf. Die Außenkontur des Griffabschnitts 26 ist zur besseren Handhabung ergonomisch ausgebildet.

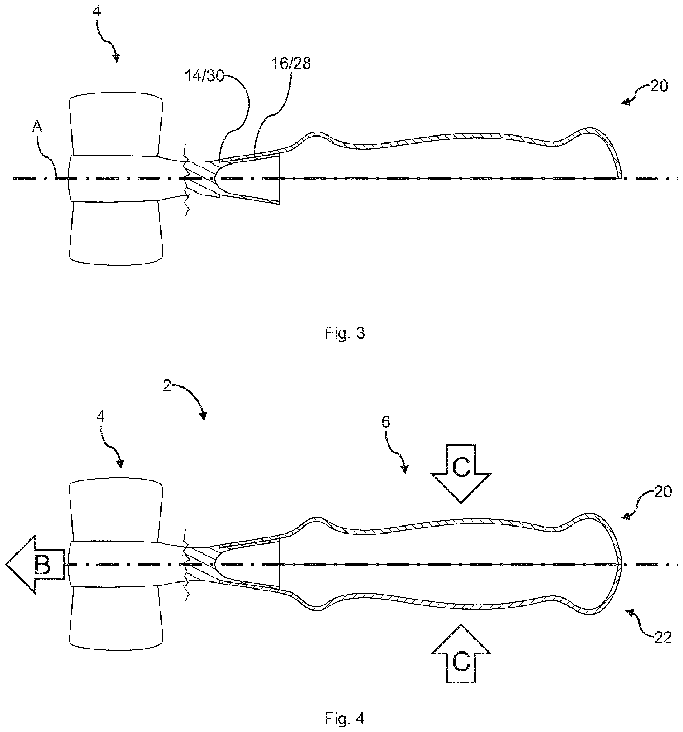

Der Verbindungsabschnitt 24 des Werkzeughefts 6 ist wie der Verbindungsabschnitt 12 des Arbeitsteils 4 ein elliptischer Hohlzylinderstumpf, wobei die Innenmaße des Verbindungsabschnitts 24 entsprechend der Außenmaße des Verbindungsabschnitts 12 gewählt sind. Das heißt, wenn, wie in Fig. 4 gezeigt, beide wannenförmigen Heftteile 20 und 22 in vorbestimmter Weise mit dem Arbeitsteil 4 zwischen sich zusammengeführt sind, dann ist der Verbindungsabschnitt 12 des Arbeitsteils 4 so in dem Verbindungsabschnitt 24 des Werkzeughefts 6 eingebettet, dass die Außenfläche des Verbindungsabschnitts 12 flächig an einer Innenfläche des Verbindungsabschnitts 24 aufliegt. Die (konische) Innenfläche des Verbindungsabschnitts 24 bildet somit eine Verengung 28 des Werkzeughefts 6.

Am distalen Ende weist das Werkzeugheft 6 eine distale Stirnfläche 30 auf.

Ausgehend von dieser Stirnfläche 30 vergrößert sich das Querschnittsaußenausmaß des Werkzeughefts 6 in proximaler Richtung über ein proximales Ende des Verbindungsabschnitts 24 hinweg bis zu einem ersten Maximalwert in einem distalen Bereich des Griffabschnitts 26, in welchem ein distaler Griffanschlag 32 ausgebildet ist.

Ausgehend von dem distalen Griffanschlag 32 verkleinert sich das Querschnittsaußenausmaß des Werkzeughefts 6 in proximaler Richtung zunächst, bleibt dann über eine Strecke, die in etwa der Durchschnittsbreite eines Handtellers entspricht, im Wesentlichen konstant und vergrößert sich schließlich wieder bis zu einem zweiten Maximalwert in einem proximalen Bereich des Griffabschnitts 26, in welchem ein proximaler Griffanschlag 34 ausgebildet ist.

Ausgehend von dem proximalen Griffanschlag 34 verkleinert sich das Querschnittsaußenausmaß des Werkzeughefts 6 in proximaler Richtung bis auf null, so dass eine proximale Stirnfläche 36 ausgebildet ist.

Die Figuren 3 bis 8 veranschaulichen die verschiedenen Stufen bei der Herstellung eines erfindungsgemäßen medizinischen Werkzeugs 2 gemäß einem erfindungsgemäßen Verfahren.

Wie in Fig. 3 gezeigt, wird zunächst das eine Heftteil 20 so mit dem Arbeitsteil 4 in Anlage gebracht, dass der entsprechende Teil der distalen Stirnfläche 30 des Werkzeughefts 6 auf dem entsprechenden Teil des Absatzes 14 des Arbeitsteils 4 aufliegt und dass der entsprechende Teil der Innenfläche des Verbindungsabschnitts 24 des Werkzeughefts 6 auf dem entsprechenden Teil der Außenfläche des Verbindungsabschnitts 12 des Arbeitsteils 4 aufliegt.

Anschließend wird das andere Heftteil 22 so mit dem aus dem Heftteil 20 und dem Arbeitsteil 4 bestehenden Verbund in Anlage gebracht, dass es mit dem Heftteil 20 den abgeplatteten Rotationskörper des Werkzeughefts 6 komplettiert und den Verbindungsabschnitt 12 des Arbeitsteils 4 einfasst. Die Einfassung des Verbindungsabschnitts 12 erfolgt dabei derart, dass das Arbeitsteil 4 relativ zum Werkzeugheft 6 nicht in axialer Richtung, d.h. nicht entlang der Haupterstreckungsachse A des Werkzeugs 2, bewegt werden kann (siehe Pfeil B in den Figuren 4 und 5), wenn die beiden Heftteile 20 und 22 aneinander gedrückt bzw. gepresst werden (siehe Pfeile C in Fig. 4).

In diesem Zustand, in dem die beiden Heftteile 20 und 22 aneinander gepresst werden, werden die beiden Heftteile 20 und 22 mit dem Arbeitsteil 4 verschweißt. Dabei werden zunächst die beiden Punkte an der distalen Stirnseite 30 des Werkzeughefts 6 mit dem Absatz 14 des Arbeitsteils 4 verschweißt, an welchen auch die beiden Heftteile 20 und 24 aneinanderstoßen (siehe Punkt 38 in Fig. 6). Dadurch werden die beiden Heftteile 20 und 22 mit dem Arbeitsteil 4 bereits mit einem einzigen Schweißpunkt miteinander stoffschlüssig verbunden. Anschließend werden die restlichen Bereiche des Absatzes 14 mit den entsprechenden Teilen der distalen Stirnseite 30 der Heftteile 20 und 22 verschweißt (siehe Schweißnaht 40).

Wie in Fig. 7 gezeigt, werden dann die restlichen Anlageflächen zwischen den beiden Heftteilen 20 und 22 verschweißt (siehe Schweißnaht 42). Im Bereich der Verbindungsabschnitte 12 und 24 werden die Heftteile 20 und 22 derart miteinander verschweißt, dass wie im Punkt 38 alle drei Teile (Arbeitsteil 4, Heftteile 20 und 22) des medizinischen Werkzeugs 2 stoffschlüssig miteinander verbunden sind (siehe Bereich zwischen den Punkten 38 und 44 in Fig. 7).

Wie in Fig. 8 angedeutet, werden abschließend die überstehenden Wülste der Schweißnähte 40 und 42 entfernt, so dass das medizinische Werkzeug 2 eine möglichst geringe Außenoberfläche für Verunreinigungen aufweist.

Die in den Figuren 1 bis 8 gezeigte und oben beschriebene Ausführungsform des erfindungsgemäßen medizinischen Werkzeugs stellt lediglich eine mögliche Umsetzung der beanspruchten Erfindung dar.

Beispielsweise könnten die Querschnittsaußenausmaße des Schaftabschnitts 10, der Verbindungsabschnitte 12 und 24 sowie des Griffabschnitts 26 auch kreisrund statt elliptisch sein.

Die Innenfläche des Verbindungsabschnitts 24 des Werkezughefts 6 und entsprechend die Außenfläche des Verbindungsabschnitts 12 des Arbeitsteils 4 müssen nicht konisch sein. Sie können also nicht nur so ausgestaltet sein, dass sich deren Querschnittsinnenausmaß bzw. deren Querschnittsaußenausmaß in proximaler Richtung kontinuierlich vergrößert, sondern können auch so, dass sie nicht-kontinuierliche, beispielsweise exponentielle oder quadratische Querschnittsausmaßänderungen aufweisen.

### Bezugszeichenliste

- 2: medizinisches Werkzeug
- 4: Arbeitsteil
- 6: Werkzeugheft
- 8: Funktionsabschnitt
- 10: Schaftabschnitt
- 12: Verbindungsabschnitt des Arbeitsteils
- 14: Absatz
- 16: Ausweitung
- 18: Aushöhlung
- 20, 22: Heftteil
- 24: Verbindungsabschnitt des Werkzeughefts
- 26: Griffabschnitt
- 28: Verengung
- 30: distale Stirnfläche
- 32: distaler Griffanschlag
- 34: proximaler Griffanschlag
- 36: proximale Stirnfläche
- 38: Schweißpunkt
- 40: Schweißnaht zwischen Arbeitsteil und Werkzeugheft
- 42: Schweißpunkt
- 44: Schweißnaht zwischen Heftteile

- A: Haupterstreckungsachse des Werkzeugs
- B: Zugkraft
- C: Zusammenpresskraft

## Patentansprüche

1. Medizinisches Werkzeug (2), insbesondere in Form eines klinischen Hammers, mit
einem Werkzeugheft (6) und einem in dem Werkzeugheft (6) aufgenommenen und mit diesem stoffschlüssig verbundenen Arbeitsteil (4),
wobei ein in dem Werkzeugheft (6) aufgenommener Abschnitt (12) des Arbeitsteils (4) eine Aufweitung (16) aufweist, welche zumindest im Falle eines Lösens des Stoffschlusses (40, 42) zwischen Werkzeugheft (6) und Arbeitsteil (4) zur Axialsicherung einen axialen Formschluss mit einer Verengung (28) des Werkzeughefts (6) eingeht,
**dadurch gekennzeichnet, dass**
das Werkzeugheft (6) zumindest zwei miteinander verbundene Heftteile (20, 22) aufweist und der in dem Werkzeugheft (6) aufgenommene Abschnitt (12) des Arbeitsteils (4) zwischen den zumindest zwei Heftteilen (20, 22) aufgenommen ist.

2. Medizinisches Werkzeug (2) nach Anspruch 1, **dadurch gekennzeichnet, dass** die zwei Heftteile (20, 22) stoffschlüssig miteinander verbunden sind.

3. Medizinisches Werkzeug (2) nach Anspruch 2, **dadurch gekennzeichnet, dass** in einem Abschnitt (38) des medizinischen Werkzeugs (2) der Stoffschluss (44) zwischen den zumindest zwei Heftteilen (20, 22) gleichzeitig auch den Stoffschluss (42) zwischen Werkzeugheft (6) und Arbeitsteil (4) gewährleistet.

4. Medizinisches Werkzeug (2) nach einem der Ansprüche 1 bis 3, **dadurch gekennzeichnet, dass** der in dem Werkzeugheft (6) aufgenommene Abschnitt (12) des Arbeitsteils (4) zumindest teilweise nicht rotationssymmetrisch ist und vorzugsweise einen elliptischen Querschnitt aufweist.

5. Medizinisches Werkzeug (2) nach einem der Ansprüche 1 bis 4, **dadurch gekennzeichnet, dass** die Aufweitung (16) an einem proximalen Ende des Arbeitsteils (4) vorgesehen ist.

6. Medizinisches Werkzeug (2) nach Anspruch 5, **dadurch gekennzeichnet, dass** an dem proximalen Ende des proximalen Abschnitts des Arbeitsteils eine axiale Aushöhlung (18) vorgesehen ist.

7. Medizinisches Werkzeug (2) nach einem der Ansprüche 1 bis 6, **dadurch gekennzeichnet, dass** an einem distalen Ende des in dem Werkzeugheft (6) aufgenommenen Abschnitts (12) des Arbeitsteils (4) eine weitere Aufweitung vorgesehen ist, welche einen Absatz (14) bildet, der mit einem distalen Ende des Werkzeughefts (6) stoffschlüssig verbunden ist und der im Falle eines Lösens des Stoffschlusses (40, 42) zwischen Werkzeugheft (6) und Arbeitsteil (4) eine zusätzliche formschlüssige Axialsicherung gewährleistet, vorzugweise in einer Richtung entgegengesetzt der Richtung, in welche die Aufweitung (16) des in dem Werkzeugheft (6) aufgenommenen Abschnitts (12) des Arbeitsteils (4) axial sichert.

8. Medizinisches Werkzeug (2) nach einem der Ansprüche 1 bis 7, **dadurch gekennzeichnet, dass** zwischen der Verengung (28) des Werkzeughefts (6) und der einem axialen Formschluss dienenden Aufweitung (16) des Arbeitsteils ein axiales Spiel vorgesehen ist, damit ein Lösen des Stoffschlusses zwischen Werkzeugheft (6) und Arbeitsteil (4) gegebenenfalls bemerkbar ist.

9. Verfahren zur Herstellung eines medizinischen Werkzeugs (2) mit den Schritten
- formschlüssiges Verbinden eines Werkzeughefts (6) mit einem Arbeitsteil (4) und
- stoffschlüssiges Verbinden des Werkzeughefts (6) mit dem Arbeitsteil (4),
**dadurch gekennzeichnet, dass**
der Formschluss zwischen Werkzeugheft (6) und Arbeitsteil (4) durch Einlegen eines mit einer Aufweitung (16) versehenen Abschnitts (12) des Arbeitsteils (4) zwischen zwei eine Verengung (28) bildende Heftteile (20, 22) des Werkzeughefts (6) bewerkstelligt wird und
anschließend die zumindest zwei Heftteile (20, 22) miteinander stoffschlüssig verbunden werden.

10. Verfahren nach Anspruch 9, **dadurch gekennzeichnet, dass** der Stoffschluss zwischen Werkzeugheft (6) und Arbeitsteil (4) und der Stoffschluss zwischen den zumindest zwei Heftteilen (20, 22) des Werkzeughefts (6) zumindest teilweise gleichzeitig bewirkt werden.

## Claims

1. A medical tool (2), in particular in the form of a clinical hammer, comprising
a tool handle (6) and a working part (4) received in the tool handle (6) and firmly bonded thereto,
wherein a portion (12) of the working part (4) received in the tool handle (6) has a widening (16) which, at least in the event of loosening of the firm bonding (40, 42) between the tool handle (6) and the working part (4), enters into an axial form fit with a narrowing (28) of the tool handle (6) for axial securing,
**characterized in that**
the tool handle (6) has at least two handle parts (20, 22) connected to each other, and the portion (12) of the working part (4) received in the tool handle (6) is received between the at least two handle parts (20, 22).

2. The medical tool (2) according to claim 1, **characterized in that** the two handle parts (20, 22) are firmly bonded to each other.

3. The medical tool (2) according to claim 2, **characterized in that** in a portion (38) of the medical tool (2), the firm bonding (44) between the at least two handle parts (20, 22) simultaneously also ensures the firm bonding (42) between the tool handle (6) and the working part (4).

4. The medical tool (2) according to one of claims 1 to 3, **characterized in that** the portion (12) of the working part (4) received in the tool handle (6) is at least partially non-rotationally symmetrical and preferably has an elliptical cross-section.

5. The medical tool (2) according to one of claims 1 to 4, **characterized in that** the widening (16) is provided at a proximal end of the working part (4).

6. The medical tool (2) according to claim 5, **characterized in that** an axial cavity (18) is provided at the proximal end of the proximal portion of the working part.

7. The medical tool (2) according to one of claims 1 to 6, **characterized in that** at a distal end of the portion (12) of the working part (4) received in the tool handle (6), a further widening is provided which forms a shoulder (14) which is firmly bonded to a distal end of the tool handle (6) and which, in the event of loosening of the firm bonding (40, 42) between the tool handle (6) and the working part (4), ensures additional form-fitting axial securing, preferably in a direction opposite to the direction in which the widening (16) of the portion (12) of the working part (4) received in the tool handle (6) secures it axially.

8. The medical tool (2) according to one of claims 1 to 7, **characterized in that** an axial clearance is provided between the narrowing (28) of the tool handle (6) and the widening (16) of the working part serving for an axial form fit, so that loosening of the firm bonding between tool handle (6) and working part (4) is noticeable if required.

9. A method of manufacturing a medical tool (2) comprising the steps of
- form-fittingly connecting a tool handle (6) with a working part (4), and
- connecting the tool handle (6) to the working part (4) in a firmly bonded manner,
**characterized in that**
the form fit between tool handle (6) and working part (4) is achieved by inserting a portion (12) of the working part (4) provided with a widening (16) between two handle parts (20, 22) of the tool handle (6) forming a narrowing (28), and
subsequently the at least two handle parts (20, 22) are firmly bonded to each other.

10. The method according to claim 9, **characterized in that** the firm bonding between tool handle (6) and working part (4) and the firm bonding between the at least two handle parts (20, 22) of the tool handle (6) are effected at least partially simultaneously.

## Revendications

1. Outil médical (2), en particulier sous forme de marteau clinique, avec
un manche d'outil (6) et une pièce de travail (4) logée dans le manche d'outil (6) et connectée à celui-ci par complémentarité de matière,
dans lequel une section (12) de la pièce de travail (4) logée dans le manche d'outil (6) présente un élargissement (16), lequel, au moins dans le cas d'un relâchement de la complémentarité de matière (40, 42) entre le manche d'outil (6) et la pièce de travail (4), forme une complémentarité de forme axiale avec un rétrécissement (28) du manche d'outil (6) pour la fixation axiale,
**caractérisé en ce que**
le manche d'outil (6) présente au moins deux pièces de manche (20, 22) connectées entre elles et la section (12) de la pièce de travail (4) logée dans le manche d'outil (6) est logée entre les au moins deux pièces de manche (20, 22).

2. Outil médical (2) selon la revendication 1, **caractérisé en ce que** les deux pièces de manche (20, 22) sont connectées l'une à l'autre par complémentarité de matière.

3. Outil médical (2) selon la revendication 2, **caractérisé en ce que** dans une section (38) de l'outil médical (2), la complémentarité de matière (44) entre les au moins deux pièces de manche (20, 22) assure aussi simultanément la complémentarité de matière (42) entre le manche d'outil (6) et la pièce de travail (4).

4. Outil médical (2) selon l'une quelconque des revendications 1 à 3, **caractérisé en ce que** la section (12) de la pièce de travail (4) logée dans le manche d'outil (6) n'est au moins partiellement pas à rotation symétrique et présente de préférence une section transversale elliptique.

5. Outil médical (2) selon l'une quelconque des revendications 1 à 4, **caractérisé en ce que** l'élargissement (16) est prévu à une extrémité proximale de la pièce de travail (4).

6. Outil médical (2) selon la revendication 5, **caractérisé en ce qu'**une cavité axiale (18) est prévue à l'extrémité proximale de la section proximale de la pièce de travail.

7. Outil médical (2) selon l'une quelconque des revendications 1 à 6, **caractérisé en ce qu'**un élargissement supplémentaire est prévu à une extrémité distale de la section (12) de la pièce de travail (4) logée dans le manche d'outil (6), lequel élargissement forme un épaulement (14), qui est connecté par complémentarité de matière à une extrémité distale du manche d'outil (6) et qui, dans le cas d'un relâchement de la complémentarité de matière (40, 42) entre le manche d'outil (6) et la pièce de travail (4), assure une fixation axiale supplémentaire par complémentarité de forme, de préférence dans une direction opposée à la direction dans laquelle l'élargissement (16) de la section (12) de la pièce de travail (4) logée dans le manche d'outil (6) la fixe axialement.

8. Outil médical (2) selon l'une quelconque des revendications 1 à 7, **caractérisé en ce qu'**un jeu axial est prévu entre le rétrécissement (28) du manche d'outil (6) et l'élargissement (16) de la pièce de travail servant à une complémentarité de forme axiale, de sorte qu'un relâchement de la complémentarité de matière entre le manche d'outil (6) et la pièce de travail (4) peut être perceptible le cas échéant.

9. Procédé de fabrication d'un outil médical (2) avec les étapes de
- liaison par complémentarité de forme d'un manche d'outil (6) avec une pièce de travail (4) et
- liaison par complémentarité de matière du manche d'outil (6) avec la pièce de travail (4), **caractérisé en ce que**
la complémentarité de forme entre le manche d'outil (6) et la pièce de travail (4) par pose avec une section (12) de la pièce de travail (4) dotée d'un élargissement (16) entre deux pièces de manche (20, 22) du manche d'outil (6) formant un rétrécissement (28) est réalisée et
les au moins deux pièces de manche (20, 22) sont ensuite connectées ensemble par complémentarité de matière.

10. Procédé selon la revendication 9, **caractérisé en ce que** la complémentarité de matière entre le manche d'outil (6) et la pièce de travail (4) et la complémentarité de matière entre les au moins deux pièces de manche (20, 22) du manche d'outil (6) sont réalisées au moins partiellement simultanément.
